(19) Europäisches Patentamt
European Patent Office
Office européen des brevets

(11) Publication number: **0 260 090**
**A2**

(12) # EUROPEAN PATENT APPLICATION

(21) Application number: **87307885.1**

(22) Date of filing: **07.09.87**

(51) Int. Cl.[4]: **C 12 N 15/00**
A 61 K 39/29, C 12 N 7/00

(30) Priority: **08.09.86 GB 8621578**
**23.09.86 GB 8622883**

(43) Date of publication of application:
**16.03.88 Bulletin 88/11**

(84) Designated Contracting States:
**AT BE CH DE ES FR GB GR IT LI LU NL SE**

(71) Applicant: **Bishop, David H.L.**
**15 Rawlinson Road**
**Oxford OX2 6UE (GB)**

**Kang, Chil-Yong**
**15 Rawlinson Road**
**Oxford OX2 6UE (GB)**

(72) Inventor: **Bishop, David H.L.**
**15 Rawlinson Road**
**Oxford OX2 6UE (GB)**

**Kang, Chil-Yong**
**15 Rawlinson Road**
**Oxford OX2 6UE (GB)**

(74) Representative: **Ritter, Stephen David et al**
**Mathys & Squire 10 Fleet Street**
**London EC4Y 1AY (GB)**

The microorganism(s) has (have) been deposited with American Type Culture Collection under number(s) 67203, 67220, 67221.

(54) **Expression of hepatitis b viral antigens from recombinant baculovirus vectors.**

(57) This invention described the expression of human hepatitis B virus (HBV) antigens in insects and in cultured insect cells. Permissive insects and cells are infected with recombinant baculoviruses that have has the requisite human HBV genes inserted into the baculovirus genome under the control of the baculovirus polyhedrin gene promoter and in lieu of the initial (5′) coding sequences of the viral polyhedrin protein.

EP 0 260 090 A2

## Description

### EXPRESSION OF HEPATITIS B VIRAL ANTIGENS FROM RECOMBINANT BACULOVIRUS VECTORS

This invention relates to a method of producing human hepatitis B virus surface antigen (HBsAg) and the related Pre-S2 protein.

Human Hepatitits B virus HBV, a member of the hepadnavirus group, is a common cause of chronic liver disease and associated hepatocellular carcinoma throughout the world. The virus genome consists of a small circular, partially single-stranded, deoxyribonucleic acid (DNA) species that in the virus particle is associated with a viral DNA polymerase. The DNA polymerase functions to complete the viral DNA into a double-stranded form and for viral DNA replication in an infected cell. HBV has a tropism for hepatocytes. It is believed that the molecular basis for this tropism resides in the outer surface proteins of the virus particle that at some stage during the initiation of an infection reacts with cellular structures (receptors) that are present on permissive cells. The initiation of infections of cells and man can be blocked by antibodies produced in humans against the outer viral proteins. Infections of humans usually begins with an acute disease that often leads to a long term chronic condition involving a persistent virus infection with high concentrations of viral antigen (some in the form of the non-infectious so-called 22 nm particles or Australia antigen) and 42 nm infectious virus ('Dane particles') cocirculating in the patient's blood for extended periods. Virus and viral antigen are also found in other body fluids (semen, saliva, nasopharyngeal secretions, menstrual fluids, ascitic fluids, bile, tears, milk, cerebrospinal fluids, etc) and may, with circumstance, be involved in virus transmission. Both the acute and chronic phases of infections in human 'carriers' are important in the epidemiology of the disease.

The so-called 22 nm particle (range 15-25 nm) which is in the form of a lipid enveloped small spherical structure, is produced as a side produce of active virus infections of permissive cells. It is principally composed of the outer surface proteins (antigens) of the virus (principally HBsAg as well as some Pre-S2) that are embedded in the lipid envelope. Other virus induced structures that contain these antigens are the filamentous or rod-shaped structures (some 22 nm wide but several hundred nanometers long) that are also found in patients' blood. The HBsAg is a viral coded glycoprotein (a protein with up to 3 carbohydrate side chains) of which the protein component is specified by the virus genetic information and the carbohydrate components are specified by the machinery of the cells in which the virus grew. The lipid components of the 22 nm and other particles, like that of the infectious virus, are derived from the lipid membrane of the host cell in which the virus grew. Thus both the lipid and carbohydrate components are specified by the cell. The sequence of the HBsAg protein, its inherent antigenic structures, its attachment sites for the carbohydrate side-chains and the part of the HBsAg protein which is embedded in the lipid are specified by the virus genetic sequence. The spheical 42 nm infectious virus particle consists of an inner core of DNA and associated DNA polymerase including the core antigen (HBcAg) and an 'e' antigen (HBeAg) surrounded by an outer 7 nm shell composed of lipid, HBsAg and some Pre-S2 protein. The Pre-S2 protein is a minor component of viruses and other virus induced structures and may represent an incompletely processed protein precursor to HBsAg since it consists of the whole HBsAg sequence and approximately 55 extra amino acids at the amino terminus end ('upstream') of HBsAg.

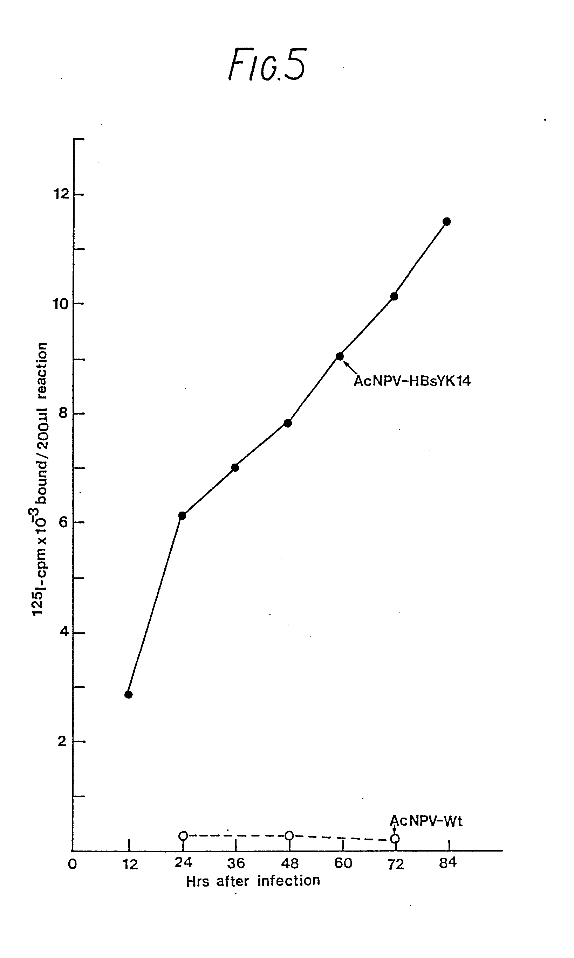

A genetic map including the locations of restriction enzyme sites (Bam HI, Acc I, etc) for approximately 3200 base long HBV (Robinson, 1985) is shown in Figure 5 on page 1392 of "Virology" ed. BN Fields, Raven Press, NW. The DNA has 4 open reading frames (ORF) in the complete (long) strand of the partially doubled stranded viral DNA. An ORF consists of DNA nucleotide sequences that from the complementary messenger ribonucleic acid (mRNA) sequence may be translated into a contiguous sequence of amino acids (protein). ORF-2 contains the sequence specifying the approximate 25,000 Dalton HBsAg (approximately 200 amino acids) that is in phase and downstream of a 163 amino acid 'Pre-S' sequence. How HBsAg and the Pre-S2 proteins are derived from the ORF-2 coding sequence is not certain. ORF-3 codes for the major core protein corresponding to HBcAg (190-220 amino acids). The identities of the gene products of ORF-1 (probably the viral polymerase) and ORF-4 are not certain.

Infection of man by HBV usually involves the intimate (often percutaneous) transfer of serum and serum containing materials between individuals (hence the alternate name of the disease 'serum hepatitis'). The disease is prevalent particularly in developing countries such as China, Oceania, South Pacific, Africa, the Middle East and some parts of South America and India with very high rates (5-20%) in certain countries of South East Asia. However the disease also occurs in other parts of the world particularly among specific ethnic groups, close contacts of chronic carriers, sexual contacts of individuals with acute hepatitis B, neonates of infected mothers, sexually promiscuous individuals, illicit drug users who share needles haemophiliacs receiving blood derived products, haemodialysis patients and their contacts, as well as health care workers (dentists, nursing staff, surgeons, clinical laboratory staff). The association of hepatocellular carcinoma and hepatitis B virus infection is believed to be due to an initial virus infection that from a chronic antigenemia leads to chronic hepatitis, cirrhosis then hepatoma development.

Diagnosis of infection by hepatitis B virus is usually initiated by clinical description of the disease followed by biochemical tests (transaminase, bilirubin, etc) that monitor the disturbed metabolism of the patient. Following the initiation of hepatitis B virus infection antibodies are produced to HBsAg, HBcAg and HBeAg allowing diagnosis of infections to be made either by the indentification of the presence of viral antigens (including virus) in blood (faceces, etc: 1-6 months; radioimmune assays [RIA], enzyme-linked immunosorbent assays [ELISA], immunoelectrophoresis, haemagglutination, etc), or by the identification of the presence of virus specific anitbodies (e.g., immunoglobulin M antiHBcAg early [from 2-8 months]; immunoglobulin G antiHBeAg [from 4-10 months] and immunoglobulin G antiHBcAg [from 5-6 months and throughout life] by RIA, ELISA, immunoelectrophoresis, haemagglutination (etc).

Although the details are not certain, within a permissive cell the infection process involving HBV is believed to involve uncoating of the genome from the outer viral components, translocation of the viral DNA to the cell nucleus, synthesis of viral DNA to render the DNA into an essentially circular form, transcription of mRNA and complete RNA copies from viral promoters in the genome, translocation of the RNA to the cell cytoplasm, translation of the viral protein species from the mRNA species, and protein priming of DNA copies of the genome from viral RNA transcripts (reverse transcription). Eventually viral core structures are formed consisting of partially double-stranded DNA forms of the genome with associated core antigens (proteins). After translation, processing and translocation of the HBsAg (and Pre-S2) to the cell surface, envelopment of the core structures occurs at the cell surface. The liberation of virus from the surface of cells allows other cells to be infected. The liberation from infected cells of abortive structures including the 22 nm particles and filamentous forms that lack viral DNA but possess HBsAg, also occurs at the cell surface.

Hepatitis B virus surface antigen and the related Pre-S2 protein are of use in the diagnosis of the disease either as antigens for identifying viral induced antibodies in body fluids of patients or for the production of reference antibodies for identifying viral antigens in patients and other materials. HBsAg and Pre-S2 protein may also be of use in the prevention of infection by vaccination.

Present methods of obtaining HBsAg and Pre-S2 protein from active virus suffer the drawback of a shortage of raw material and also any production process involving the use of infectious virus is potentially hazardous due to the risk of infecting production workers and to the possibility of active virus passing over into the desired product. Presently available method of producing HBsAg and Pre-S2 protein by recombinant DNA techniques involving transformation of prokaryotic cells have not proved to be particularly efficient.

It has now been found that HBsAg and Pre-S2 protein may be particularly readily produced by infecting insects or insect cells with an expression vector based on a baculovirus.

Thus according to one aspect of the present invention, there is provided a process of producing a polypeptide comprising at least an antigenic portion of HBsAg or Pre-S2 protein which comprises infecting susceptible insects or insect cells with an expression vector comprising a recombinant baculovirus having a DNA segment coding for said polypetide under expressional control of a polyhedrin promotor.

The invention also includes such recombinant baculoviruses themselves. The invention thus provides products of the genes encompassing the human hepatitis B virus surface antigen (HBsAg) and related Pre-S2 protein synthesized from recombinant baculoviruses grown in insects or cultured insect cells. The recombinant baculoviruses may be produced by cotransfection of cultured insect cells with infectious baculovirus DNA with baculovirus transfer vector plasmids containing genes constructed to represent hepatitis B virus antigens and placed in lieu of the initial (5') coding sequences of the baculovirus polyhedrin gene but under the control of the residual polyhedrin promotor. Among the progeny of the cotransfections are infectious, polyhedrin-negative viruses expressing the hepatitis antigens. These viruses may be selected initially on the basis of a polyhedrin-negative phenotype and subsequently for the expression of the hepatitis B antigens when grown in insects or in cultured insect cells. The bulk of the hepatitis antigens are secreted from the recombinant virus infected cells as particles that both serologically and structurally resemble the hepatitis B 22 nm particles. They can be purified from the insect extracts or from cultured insect cell supernates by standard procedures such as differential or equilibrium gradient centrifugation and chromatography.

The fact that hepatitis antigens may be secreted from insect cells is particularly surprising having regard to previous failures to achieve secretion in other recombinant systems, e.g. in yeast cells. It is furthermore quite unexpected that assembly into particles resembling 22 nm particles takes place in an insect- or insect cell-based system.

In the present application, the following terms are defined as follows:

A plasmid (e.g., the commonly employed pUC8) is an extra-chromosomal sequence of double-stranded DNA that contains the sequences necessary for replication within a permissive cell (e.g., bacterial). It is a replicon that can be introduced into a cell by the process termed transformation.

A promoter is a sequence of DNA that is involved in facilitating the transcription of messenger RNA (mRNA) species from DNA sequences (genes that are associated with the promoter. The mRNA species are copies of one or other strand of the DNA. The mRNA can be used to direct the synthesis (translation) of proteins encoded in the DNA sequence. Transcription is catalyzed by an RNA polymerase that has the ability to recognise DNA sequences including the promoter.

A gene is a piece of DNA that by virtue of its inherent sequence (contiguous or interrupted) is responsible for the synthesis of a protein chain of amino acids.

Infection is the invasion of a permissive cell by genetic material (in the form of DNA or virus) whereby the material subsequently replicates. In the case of infection by a virus of DNA representing a virus the result may be release from those cells of infectious progeny viruses.

Transfection and cotransfection refer to experimental procedures for introducing genetic material, e.g. one or more species of DNA into cells so that the DNA can infect the cell. Where the DNA species are colinear the possibility exists for recombinational (swapping) events to occur. Transfection may be carried out by a procedure involving the precipitation of DNA with particular salts (usually calcium chloride in the presence of phosphate, dextran sulphate, etc) and the application of the resulting mixture to cells whereby some of the DNA is taken into the cells.

The production of HBsAg and Pre-S2 in accordance with the invention will be described in more detail with reference to the accompanying drawings of which:

Figure 1 illustrates the procedure by which a first recombinant transfer vector according to the invention was constructed.

Figure 2 illustrates the procedure by which a second recombinant transfer vector according to the invention was constructed.

Figure 3 shows the results of a radioimmunoassay carried out on a supernatant from an insect cell culture infected with recombinant baculoviruses according to the invention.

Figure 4 shows photomicrographs comparing such supernatants with authentic 22 nm particles isolated from blood of patients infected with HBV.

Figure 5 shows the production of HBV antigen as a function of time by insect cells infected with recombinant baculoviruses according to the invention.

The construction of recombinant baculoviruses in accordance with the invention was carried out as follows:

DNA of hepatitis B virus (adw subtype) was isolated from a patient's blood and the DNA digested into specific fragments using the restriction endonuclease enzyme Bam HI (see Figure 5 on page 1392 of "Virology" ed. BN Fields, Raven Press, NW). Restriction enzymes recognise specific nucleotide sequences in DNA and catalyze their hydrolysis at or near those sequences. Depending on the enzyme specificity, the resulting products either have 'blunt' ends in which both DNA strands of each derived DNA fragment are of equal length and neither overlaps the other at the cut sites, or they have 'sticky' ends where one strand is longer than the other. The products of digestion by restriction enzyme can be religated employing other enzymes (ligases) most easily where complementary 'sticky' ends are present (e.g., the products of Bam HI digestion of a DNA ligated back together or to the Bam HI digestion products of anther DNA). Ligation is also possible when the DNA fragments have 'blunt' ends.

Digestion of the HBV DNA with Bam H1 restrictuion enzyme yielded a fragment of approximately 1400 base pairs of the HBV DNA encompassing (see Figure 1, L strand clockwise 5′ to 3′) plus part of the 'Pre S' region, all of the HBsAg ORF-2 coding sequences and adjacent 3′ sequences. After transfer of the bulk of the necessary HBV sequence, reconstruction of the gene(s) was carried out as follows:

The Bam HI derived fragment containing the HBsAg sequences was ligated into the Bam H1 site of the bacterial plasmid pUC8 (Figure 1). The pUC8 plasmid replicates in specific bacterial cells and following the insertion of a foreign sequence such as the HBV DNA recombinant pUC8 plasmids can be replicated, cloned and selected. A recombinant pUC8-HBs was derived by these procedures, its DNA isolated and digested with Bam HI, then Acc I and the resulting large DNA fragment recovered containing the majority of the HBsAg gene (Figure 1). The fragment was then ligated to a synthetic oligonucleotide sequence to faithfully reconstruct the translation termination sequence of HBsAg, to provide restriction sites for subsequent manipulations (e.g., Bam HI sites) and to provide an end sequence representing a cut Hind III sequence ('sticky' end). After this ligation the products were religated into a pUC8 plasmid previously digested with Bam HI and Hind III (Figure 1) resulting in the formation and recovery of the recombinant plasmid pHBsYK2. DNA from this recombinant plasmid was digested with Bam HI, the fragment containing the HBV sequence isolated and ligated into the baculovirus transfer vector designated pAcRP6 Prior to ligation the transfer vector pAcRP6 was cut with Bam HI. After cloning in bacterial a recombinant plasmid transfer vector designated pAcRP6-HBsYK14 was obtained (Figure 1). Transfer vector pAcRP6 - HBsYK 14 is deposited with ATCC under accession number ATCC 67203 (12th September 1986).

The origins of the pAcRP plasmid transfer vectors are described by Possee, 5 Virus Research p. 43, 1986; Matsuura et al., 67 J. Gen. Virol p. 1515, 1986. Plasmid pAcRP6 resembles the plasmid transfer vector derived from Autographa californica nuclear polyhedrosis virus (AcNPV) as described Smith, Summers and Fraser (Mol. and Cell. Biol. 1983, Vol. 3, No. 12, pp. 2150-2165) and has a short synthetic oligonucleotide 'linker' sequence containing a Bam HI restriction enzyme recognition site in lieu of residues -8 through +175 of the polyhedrin gene, where the original polyhedrin ATG codon is counted as +1 to +3; (see also Hooft van Iddekinge, Smith and Summers 131 Virology, p 561, 1983). The Bam HI site is the only Bam HI site in the plasmid allowing unique digestion at that site by the Bam HI enzyme. Alternative baculovirus transfer vectors could have been employed. A derivative of the pAcRP6 plasmid was also constructed in which a short synthetic Bam HI - Sma I - Bam HI adapter sequence has been placed in lieu of the Bam HI linker to allow digestion of the plasmid at its unique Sma I site.

The pAcRP6-HBsYK14 recombinant transfer vector was used in cotransfections of Spodoptera frugiperda insect cell together with infectious DNA isolated from AcNPV. From the progeny viruses of the cotransfections, recombinant, polyhedrin-negative, viruses were identified by the lack of visible polyhedra in clones of infected cells, recovered and plaque cloned in cultured S. frugiperda cells. The recombinant viruses such as AcNPV - HBsYK14 were used for the expression of HBsAg in insects and in cultured insect cells.

The supernate from infected cultured insect cells was tested for HBV antigen by radioimmunoassay against $^{125}$I-labelled anit-HBV at serial dilutions from 1/1 to 1/64. The results are set forth in Figure 3 from which it can be seen that there is a linear relationship between dilution and bound antibody.

Microscopic examination (Figure 4) of cell supernate (i), clarified cell supernate (ii) 20x concentrated cell supernate (iii) and blood serum (iv) containing authentic 22 nm particles shows that insect cells infected with recombinant transfer vectors according to the invention produce particles indistinguishable from 22 nm HBV antigen.

Insect cells were infected with recombinant virus AcNPV-HBsYK14 and with the starting (untransformed) virus AcNPV. Production of HBV antigen as a function of time was measured for each and it can be seen that only the transformed vector gives a positive assay.

It has been shown that the Pre-S2 is important for the enhanced immunogenicity of the HBsAg. Therefore a second recombinant transfer vector was constructed containing the HBsAg and all the necessary upstream Pre-S2 sequences. Synthetic oligonucleotides (Figure 2) representing the upstream sequence of Pre-S2 missing from the pHBsYK2 plasmid DNA were ligated to both ends of the Bam HI derived fragment containing HBV sequences recovered from pHBsYK2 DNA. In addition to restriction enzyme sequences required for subsequent manipulations (e.g. Xho I) and the ATG translation initiation codon for the synthesis of the Pre-S2 protein, the oligonucleotides possessed appropriate terminal sequences to allow 'sticky' end ligation to the termini of Bam HI derived DNA (see Figure 2). The products of the ligation of the oligonucleotides and the Bam HI derived pHBsYK2 fragment were then ligated into the plasmid pJM119 and recombinant plasmids containing the oligonucleotide sequences recovered and shown by sequence analyses (Figure 3, pJM119-PsSYK25) to have the entire coding region of the 55 amino acid Pre-S2 upstream of the HBsAg sequence and a duplicate oligonucleotide sequence (beyond the TAA translation termination codon of the HBsAg gene) at the 3′ end but in the reverse orientation. The DNA of plasmid pJM119-PsSYK25 was digested with Xho I, the ends rendered into a blunt end form by treatment with the Klenow fragment of DNA polymerase and the appropriate deoxyribonucleoside triphosphates and the products ligated into the pAcRP6 transfer vector containing the unique Sma I site. Recombinants with the gene arrangement illustrated in Figure 2 were obtained (pAcRP6-PsSYK27).

The pAcRP6-PsSYK27 recombinant transfer vector was used in cotransfections of Spodoptera frungiperda insect cells together with infectious DNA isolated from AcNPV. From the progeny viruses of the cotransfections, recombinant, polyhedrin-negative, viruses were identified and plaque cloned in cultured S. frugiperda cells. The recombinant virused were used for the expression of Pre-S2 HBsAG in insects and in cultured insect cells.

Additional deposits of recombinant vectors and transformed virused referred to herein have been made with the American Type Culture Collection as follows:

(1) E. coli MC1061 (pAcRP6-HBsYK14) - ATTC 67220

(2) E. coli MC1061 (pAcRP6-PsSYK27) - ATTC 67221 (both filed 25th September 1986)

(3) A. californica AcNPV-PsSYK27 - VR 2157

(4) A. californica AcNPV-HBsYK14 - VR 2158 (both filed 30th December 1986)

**Claims**

1. A process of producing a polypeptide comprising at least an antigenic portion of HBsAg or Pre-S2 protein which comprises infecting susceptible insects or insect cells with an expression vector comprising a recombinant baculovirus having a DNA segment coding for said polypeptide under expressional control of a polyhedrin promotor.

2. A process according to Claim 1 wherein the recombinant baculovirus has a polyhedrin-negative phenotype.

3. A process according to Claim 1 or Claim 2 wherein the recombinant baculovirus is derived from Autographa californica nuclear polyhedrosis virus.

4. A process according to any preceding claim wherein the expression vector is formed by recombination of a recombinant transfer vector comprising said DNA segment and infectious DNA of said baculovirus.

5. A process according to Claim 4 wherein said recombination is effected by cotransfecting susceptible insects or cells with the recombinant transfer vector comprising said DNA segment and infectious DNA of said baculovirus.

6. A process according to any preceding claim wherein said DNA segment comprises the Bam HI insert of transfer vector pAcRP6-HBsYK14 (ATCC 67203).

7. A recombinant baculovirus having a DNA segment coding for at least an antigenic portion of HBsAg or Pre-S2 protein.

8. A transfer plasmid capable of reproducing in a bacterium and adapted for recombining with a baculovirus having a DNA segment coding for at least an antigenic portion of HBsAg or Pre-S2 protein.

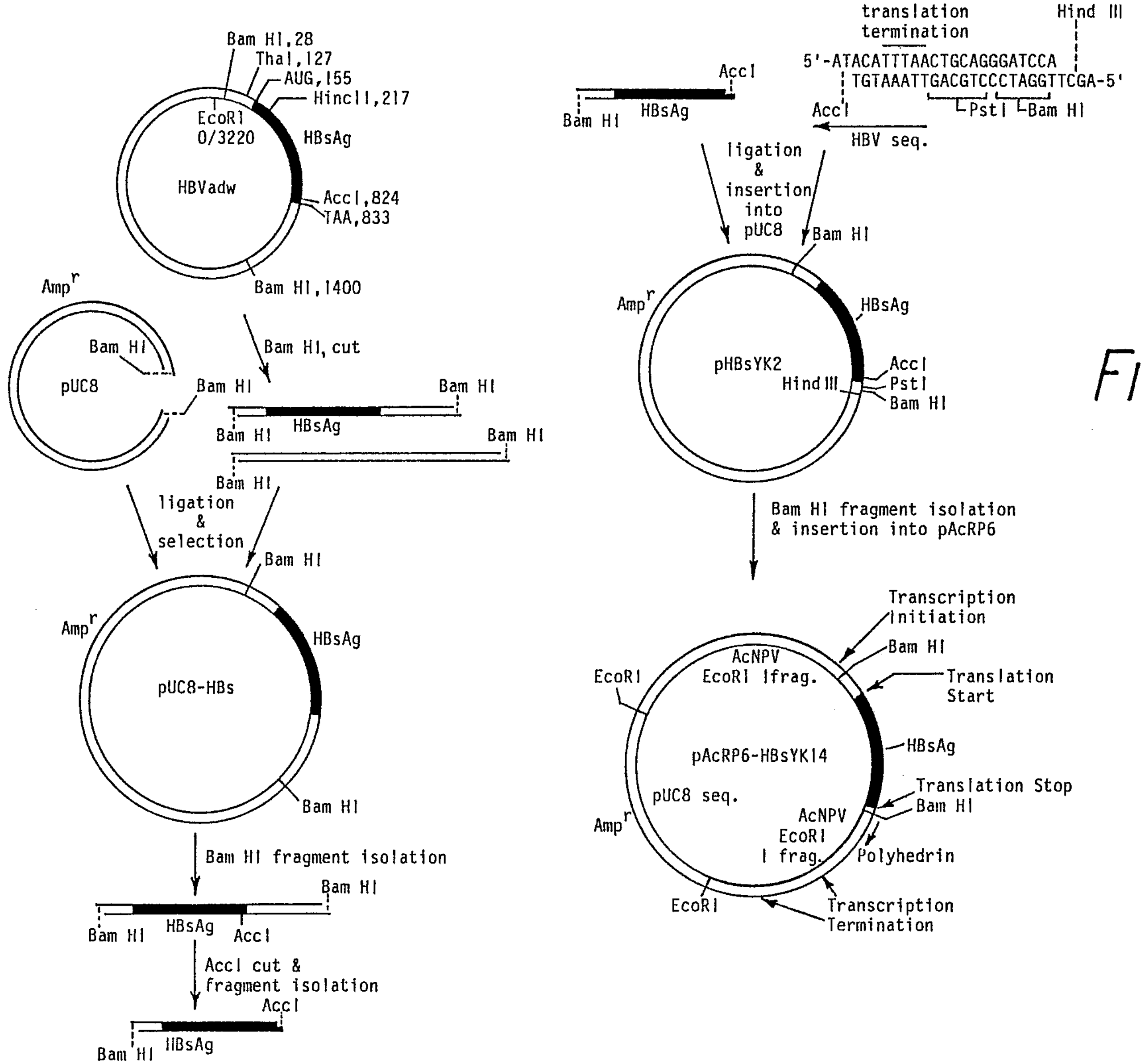

Bam HI,28
ThaI,127
AUG,155
HincII,217
EcoRI
0/3220
HBsAg
HBVadw
AccI,824
TAA,833
Bam HI,1400
Amp^r
Bam HI
pUC8
Bam HI
Bam HI, cut
Bam HI
Bam HI
HBsAg
Bam HI
Bam HI
ligation
&
selection
Bam HI
Amp^r
HBsAg
pUC8-HBs
Bam HI
Bam HI fragment isolation
Bam HI
Bam HI
HBsAg
AccI
AccI cut &
fragment isolation
AccI
Bam HI
HBsAg
AccI
Bam HI
HBsAg
translation
termination
Hind III
5'-ATACATTTAACTGCAGGGATCCA
TGTAAATTGACGTCCCTAGGTTCGA-5'
AccI
PstI
Bam HI
HBV seq.
ligation
&
insertion
into
pUC8
Bam HI
Amp^r
HBsAg
pHBsYK2
AccI
HindIII
PstI
Bam HI
Bam HI fragment isolation
& insertion into pAcRP6
Transcription
Initiation
Bam HI
Translation
Start
EcoRI
AcNPV
EcoRI Ifrag.
HBsAg
pAcRP6-HBsYK14
pUC8 seq.
Translation Stop
Bam HI
Amp^r
AcNPV
EcoRI
I frag.
Polyhedrin
EcoRI
Transcription
Termination


FIG.1

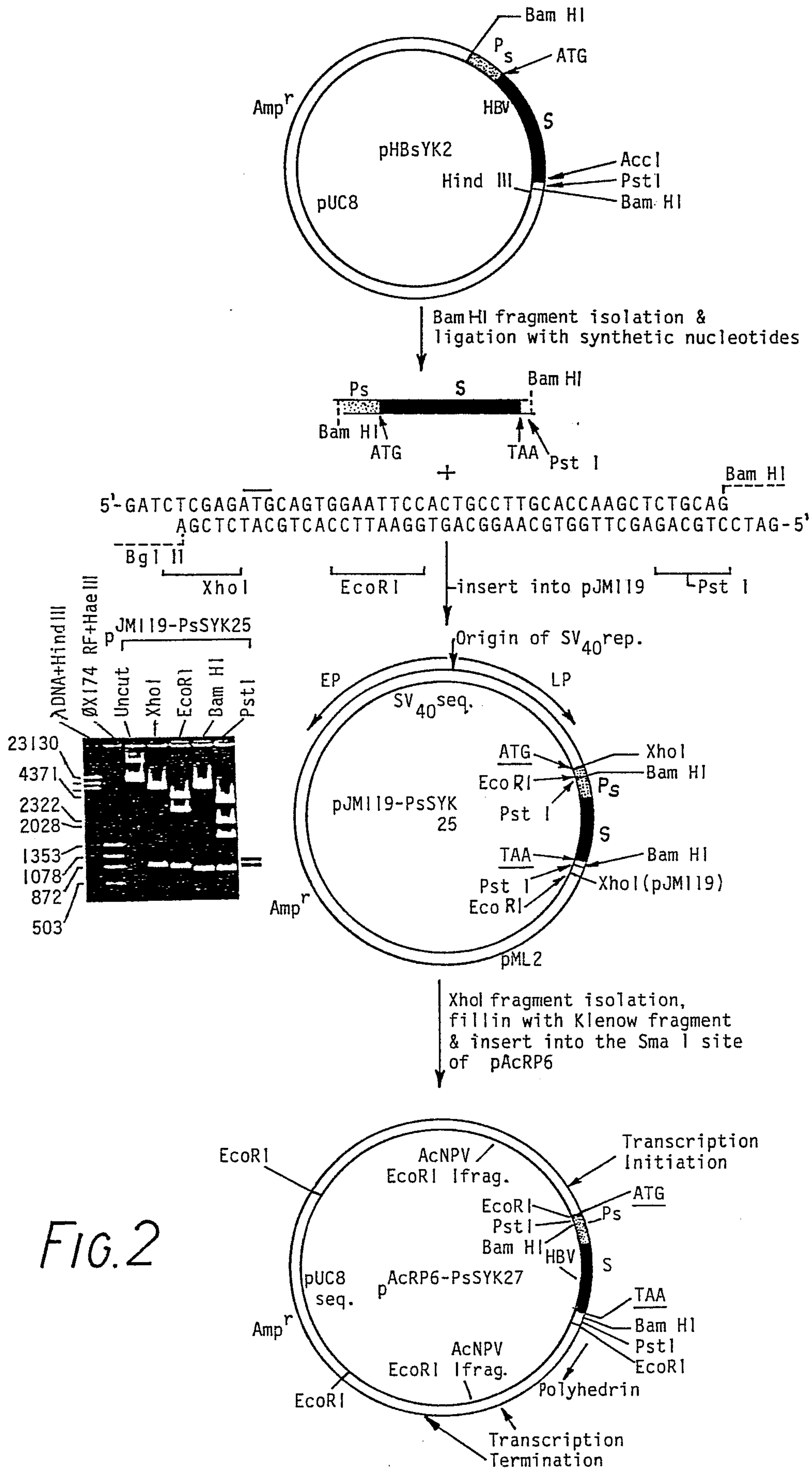
Bam HI
Ps
ATG
Amp^r
HBV
S
pHBsYK2
AccI
PstI
Hind III
Bam HI
pUC8
Bam HI fragment isolation &
ligation with synthetic nucleotides
Ps
S
Bam HI
Bam HI
ATG
TAA
Pst I
+
Bam HI
5'-GATCTCGAGATGCAGTGGAATTCCACTGCCTTGCACCAAGCTCTGCAG
AGCTCTACGTCACCTTAAGGTGACGGAACGTGGTTCGAGACGTCCTAG-5'
Bgl II
XhoI
EcoRI
insert into pJM119
Pst I
λDNA+HindIII
ØX174 RF+HaeIII
pJM119-PsSYK25
Uncut
XhoI
EcoRI
Bam HI
PstI
23130
4371
2322
2028
1353
1078
872
503
Origin of SV40 rep.
EP
LP
SV40 seq.
ATG
XhoI
Bam HI
EcoRI
Ps
pJM119-PsSYK 25
Pst I
S
TAA
Bam HI
Pst I
XhoI(pJM119)
EcoRI
Amp^r
pML2
XhoI fragment isolation,
fillin with Klenow fragment
& insert into the Sma I site
of pAcRP6
EcoRI
AcNPV
EcoRI Ifrag.
Transcription
Initiation
ATG
EcoRI
Ps
PstI
Bam HI
HBV
S
pUC8
seq.
pAcRP6-PsSYK27
TAA
Amp^r
Bam HI
PstI
AcNPV
EcoRI Ifrag.
EcoRI
Polyhedrin
EcoRI
Transcription
Termination

FIG. 2

FIG. 3

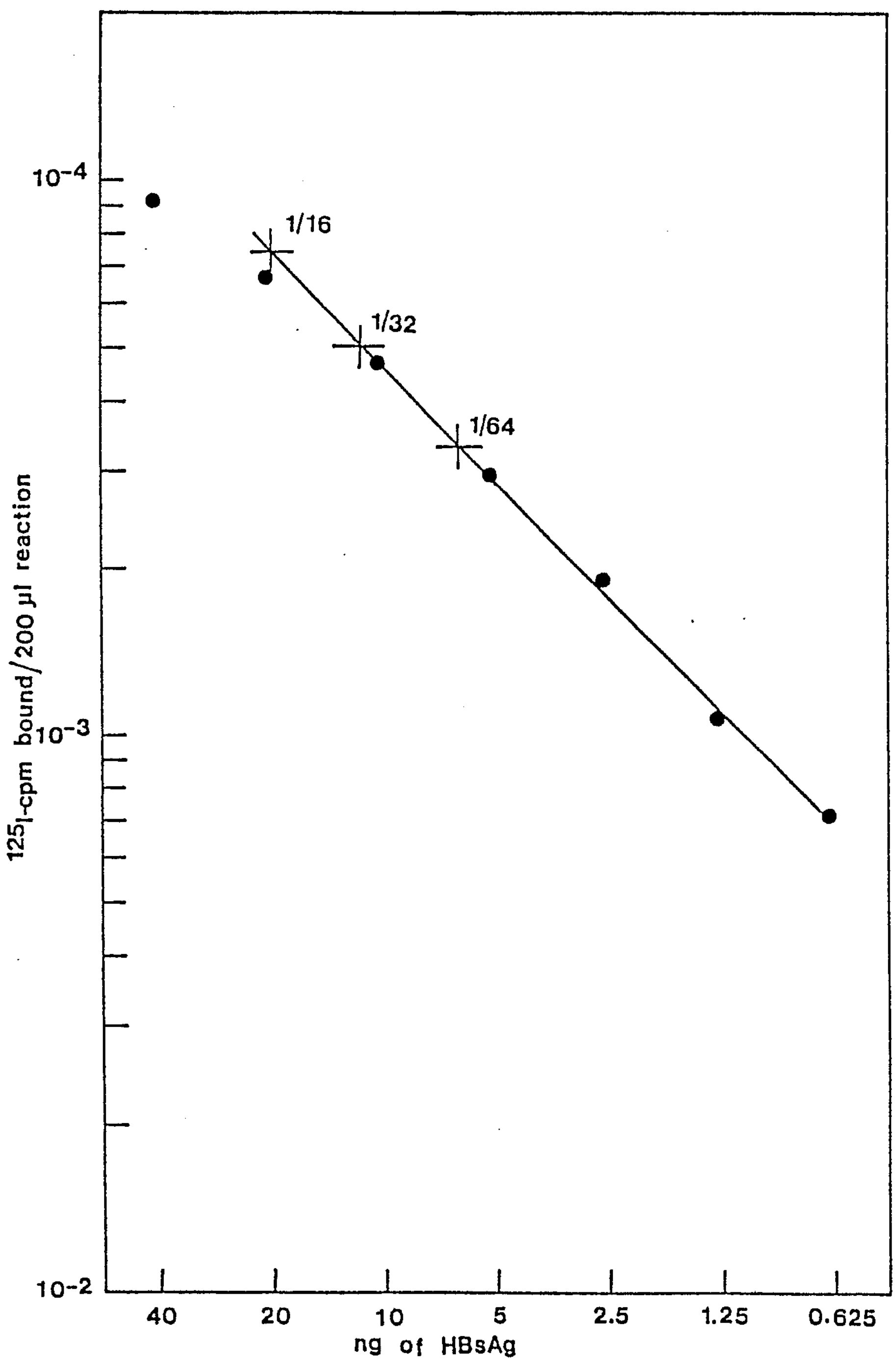

$10^{-4}$
$10^{-3}$
$10^{-2}$
1/16
1/32
1/64
$^{125}$I-cpm bound/200 µl reaction
40
20
10
5
2.5
1.25
0.625
ng of HBsAg

FIG.3 (CONT'D)

RADIOIMMUNO ASSAY

| Dilution of antigen | $HB_sAg$ standard (KGC) | | AcNPV-$HB_s$YK14 infected cell supernatant | Positive control (Abbott) | Negative control |
|---|---|---|---|---|---|
| | (cpm) | (ng) | (cpm) | (cpm) | (cpm) |
| Undiluted | 9,290 | 40 | 16,300 | 5,850 | 190 |
| 1/2 | 6,790 | 20 | 15,030 | 3,710 | 190 |
| 1/4 | 4,720 | 10 | 12,470 | 2,120 | 240 |
| 1/8 | 2,990 | 5 | 10,250 | 1,270 | 200 |
| 1/16 | 1,820 | 2.5 | 7,470 | 810 | 180 |
| 1/32 | 1,140 | 1.25 | 5,070 | NT | NT |
| 1/64 | 710 | 0.625 | 3,310 | NT | NT |

FIG.4

(i)

(ii)

(iii)

(iv)

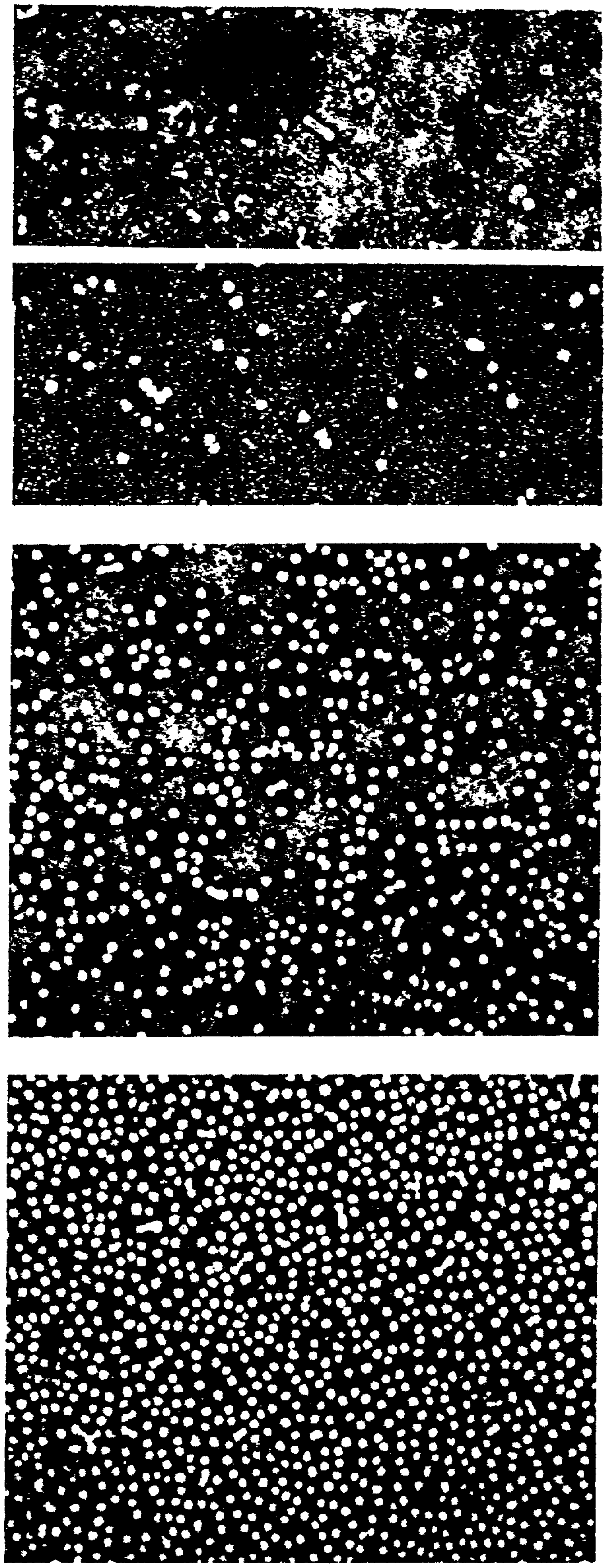

0260090

Fig.5